# EUROPEAN PATENT APPLICATION

(11) **EP 2 777 563 A1**
(43) Date of publication of application: **17.09.2014**
(21) Application number: 14159086.9
(22) Date of filing: 12.03.2014
(51) Int. Cl.: A61B 17/34, A61B 1/32, A61B 1/00

(54) **Thoracic scope with skirt and gap**

(30) Priority: 13.03.2013 US 201361779235 P; 05.03.2014 US 201414197460
(71) Applicant: Covidien LP, Mansfield, MA 02048 (US)
(72) Inventor: Luttati, John, Southington, Connecticut 06489 (US); Bettuchi, Michael J., Madison, CT 06443 (US); Bueno, Richard Roland, Madison, Connecticut 06443 (US)
(74) Representative: Soames, Candida Jane

(57) **Abstract**

A minimally invasive viewing instrument including a cannula configured and adapted to house a scope having a lens. An attachment member including a skirt member is configured and adapted to maintain the lens of the scope at a distance away from the distal end of the skirt member to minimize the potential of lens contamination. Moreover, the materials shape, and/or contours of the skirt member is configured and adapted to divert the flow of bodily fluid away from the lens, thereby reducing the potential of lens contamination.

## Description

### CROSS-REFERENCE TO RELATED APPLICATIONS

This application claims the benefit of and priority to U.S. Provisional Patent Application No. 61/779,235, filed March 13, 2013, the entire disclosure of which is incorporated by reference herein.

### BACKGROUND

### Technical Field

The present disclosure relates generally to an apparatus that inhibits the buildup of debris or moisture on the viewing portion of a minimally invasive viewing instrument. More particularly, the present disclosure relates to a thoracic scope including an apparatus that inhibits the collection of debris and/or moisture on the viewing portion of the thoracic scope. Background of Related Art

A minimally invasive surgical procedure is one in which a surgeon enters a patient's body through a small opening in the skin or through a naturally occurring opening (e.g., mouth, anus, or vagina). Such procedures have several advantages over traditional open surgeries. In particular, as compared to traditional open surgeries, minimally invasive surgical procedures result in reduced trauma and recovery time for patients. The types of minimally invasive surgeries include arthroscopic, endoscopic, laparoscopic, and thoracic surgeries.

Arthroscopy is used to diagnose and repair joint problems. Most commonly, such surgeries are performed for knee and shoulder problems. During such surgeries, a tube shaped instrument called an arthroscope is inserted into a small incision, called a portal, which is made in the side of a joint. The arthroscope includes optic fibers to transmit an image of the inside of the joint through a small camera to a video monitor in the operating room. The joint is repaired by inserting small surgical instruments through other portal incisions in the joint. These portals are so small that they usually do not require stitches.

Endoscopy is used to diagnose and treat many types of problems, including those of the ear, gallbladder, nose, and throat. During an endoscopic procedure, a flexible tube called an endoscope is inserted into a natural opening (e.g., mouth, anus, or vagina) in the patient's body or through small incisions. The endoscope includes a fiber-optic light and a video camera at its tip that transmits an image of the inside of the body to a video monitor in the operating room. If tissue looks abnormal, small instruments can be inserted through the endoscope to remove or sample the tissue.

Laparoscopy is used to diagnose and treat many types of abdominal problems, including some cancers, obstetric/gynecological problems, and urological problems. A laparoscope is a small telescope that is inserted into the abdomen through a small incision. Using a laparoscope, the surgeon is able to look at structures within the abdomen, including the adrenal glands, appendix, gallbladder, intestines, kidneys, liver, pancreas, spleen, stomach, and reproductive organs. Problems can be repaired by inserting tiny surgical instruments through other incisions in the abdomen.

Thoracoscopy is a surgery of the chest that is performed with a thoracoscope (small video-scope) using small incision and special instruments to minimize trauma. Typically, multiple small incisions are made. One keyhole-size incision accommodates the thoracoscope and additional small incisions are made for specialized surgical tools.

Common to each procedure is the use of a scope to facilitate visualization of the internal body structures. During use, debris, .e.g., organic matter and moisture, may be deposited on the viewing portion, e.g., lens, of the minimally invasive instrument. The buildup of debris on the lens impairs visualization of the surgical site. Often, removal and cleaning of the scope is required. The removal of the scope increases the time required to complete the procedure. Since an increase in time required to complete the procedure may result in additional trauma and discomfort to the patient, a continuing need exists for improved devices to clean the viewing portions of minimally invasive instruments.

### SUMMARY

Disclosed herein is a surgical instrument for use in minimally invasive surgery. The surgical instrument includes a cannula including a proximal end and a distal end. Within the cannula, a viewing instrument, e.g., an endoscope or a laparoscope, is secured. At the proximal end of the cannula, an eyepiece may be positioned to facilitate viewing through the viewing instrument. A skirt member having a lumen is coupled to the distal end of the cannula. Within the skirt member, a ring ensures that a gap exists between the distal end of the skirt member and the distal end of the cannula. This gap ensures that the lens of the viewing instrument is separated from the distal end of the skirt member. In addition, the lumen may be a relatively narrow channel to facilitate a pressure gradient that inhibits the flow of fluid up through the channel toward the lens.

Moreover, the shape, contour, texture, and/or configuration of the skirt member facilitates the diversion of blood and/or other fluids away from the distal end of the skirt member. For example, the skirt member may have a rounded, elliptical or bell shape and may include grooves or channels that are configured and adapted to divert the blood flow away from the distal end of the skirt member.

In an embodiment, the minimally invasive viewing instrument may include a cannula including a passage longitudinally disposed therethrough, the cannula including a distal end, a viewing instrument including a lens, the viewing instrument positioned within the passage of the cannula, and an attachment member configured and adapted to be operatively coupled to the distal end of the cannula.

The attachment member may include a skirt member including a proximal end, a distal end, and a longitudinally extending through lumen adapted and configured to receive the cannula therein. A ring may be positioned within the lumen to inhibit translation of the cannula beyond a given distance within the lumen, thereby maintaining a gap between the lens and the distal end of the skirt member.

Moreover, the skirt member may be formed from a material that repels fluid. In embodiments, the surface of the skirt member may have a texture and/or contour that diverts and/or propels fluid in a particular direction when passed across the surface (e.g. wicking). For example, the skirt member may have a surface that defines a plurality of channels to propel and/or divert fluid away from the distal end of the skirt member.

In another aspect of the disclosure, a method for viewing a body cavity is disclosed. The method includes accessing an opening in a tissue layer, inserting a cannula within the opening, coupling an attachment member to a distal end of the cannula, and securing a viewing instrument within a passage of the cannula. The method may include any of the cannulas, attachment members, and viewing instruments disclosed herein.

The method may further include rotating the attachment member to divert fluid away from a distal end of the attachment member. The method may further include creating an opening in a tissue layer.

These and other features of the present disclosure will be more fully described with reference to the appended figures.

### BRIEF DESCRIPTION OF THE DRAWINGS

By way of description only, embodiments of the present disclosure will be described herein with reference to the accompanying drawings, in which:
Fig. 1 is a side view of a minimally invasive viewing instrument including an attachment member shown placed within an incision;
Fig. 2 is a perspective view of the skirt member of Fig. 1;
Fig. 3 is a perspective view of another embodiment of a attachment member; and
Fig. 4 is a side view of the minimally invasive viewing instrument shown with yet another embodiment of a attachment member and placed within an incision.

### DETAILED DESCRIPTION

Particular embodiments of the present disclosure will be described with reference to the accompanying drawings. In the figures and in the description that follow, in which like reference numerals identify similar or identical elements, the term "proximal" will refer to the end of the device that is closest to the operator during use, while the term "distal" will refer to the end that is farthest from the operator during use.

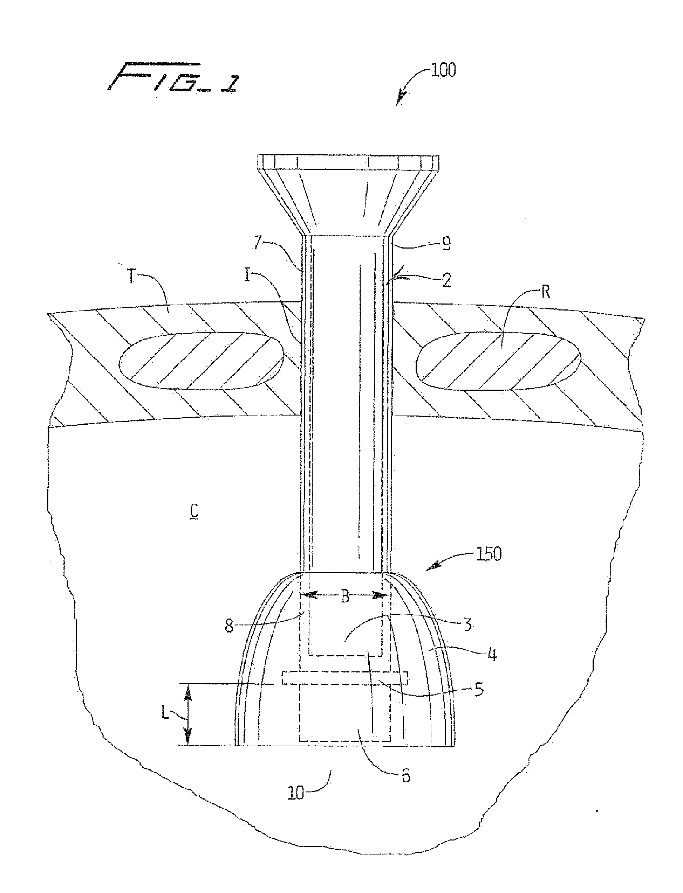

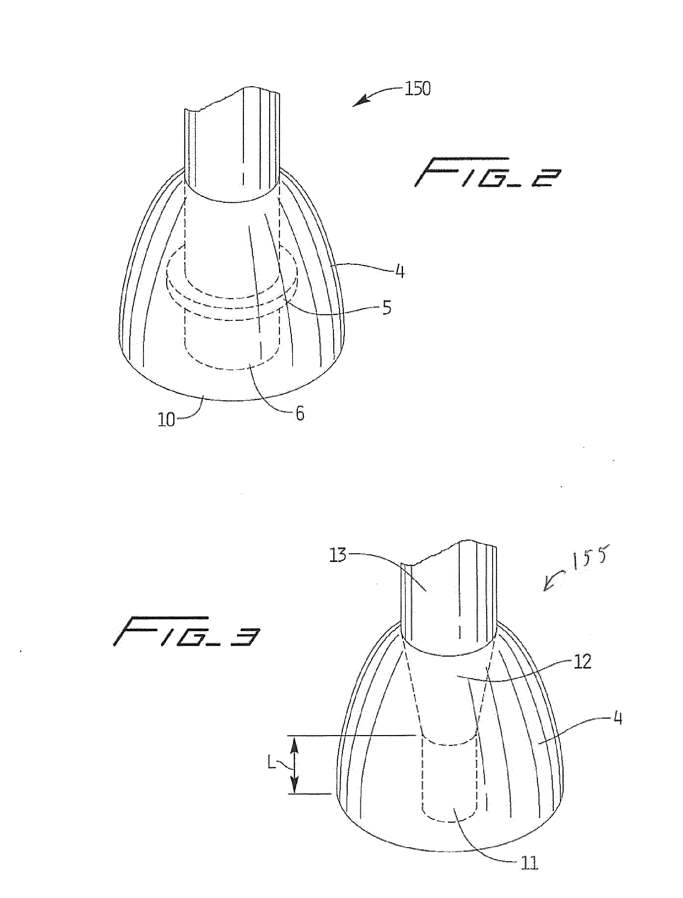

A minimally invasive viewing instrument 100 will now be described with reference to Figs. 1 and 2. The instrument 100 is configured and adapted to be inserted into an incision or into a naturally occurring body orifice to gain access to a body cavity. As shown in Fig. 1, the instrument 100 may be inserted into an incision "I" between tissue portions "T" between ribs "R" to access the thoracic cavity "C".

The instrument 100 includes a cannula 2 having a passage within which is a scope 7 having a lens 3. At a proximal end 9 of the cannula 2, an eyepiece may be operatively coupled to the cannula 2 to facilitate viewing through the scope 7. The scope may also or alternatively be operatively coupled to a remote monitor (not shown) by wired or wireless means. Although the instrument 100 is depicted and described as having a lens, any suitable viewing device may be coupled to the instrument 100.

An attachment member 150 (Figs. 1 and 2) is configured and adapted to be operatively coupled to a distal end 8 of the cannula 2. The attachment member 150 includes a skirt member 4 including a longitudinally extending through lumen 6. The attachment member 150 may be operatively coupled to the distal end 8 of the cannula 2 by placing the distal end 8 of the cannula 2 within the lumen 6 in a permanent or a releasable connection. Means of coupling the attachment member 150 to the distal end 8 of the cannula 8 include, but are not limited to, frictional, adhesive, and/or magnetic means. Other means include snap-fit or other well-known types of coupling such as bayonet.

Moreover, the attachment member 150 may be formed from a semi-rigid or a compressible material that exerts a biasing force "B" against the cannula 2 that is inserted into and extends longitudinally through lumen 6 of the attachment member 150, thereby forming a substantial seal about the cannula 2. The biasing force "B" may also serve to inhibit translation of the cannula 2 through the lumen when not manually manipulated by an operator to adjust placement of the attachment member 150. A member, such as an O-ring, or a sealant may also be placed against between cannula 2 and lumen 6, for example to aid in forming a substantial seal about the cannula 2 or inhibit translation of cannula 2 through the lumen 6.

To further inhibit translation of the cannula 2 through the lumen 6, within the lumen 6 may be a ring 5 that is configured and adapted to engage or block the distal translation of the cannula 2 through the lumen 6. In particular, the ring 5 may frictionally engage the distal end 8 of the cannula 2, thereby inhibiting distal translation of the cannula 2 farther through the lumen 6. By inhibiting the translation of the cannula 2 through the lumen 6, the lens 3 that is substantially disposed within the cannula 2 is maintained at a distance or gap "L" away from the distal end 10 of the skirt member 4.

Maintaining the gap between the lens 3 and the distal end 10 of the skirt member 4 may be accomplished by means other than the use of the ring 5. In other embodiments of attachment members, the lens 3 of the scope 7 disposed within cannula 2 is maintained at a distance "L" from the distal end of an attachment member without the use of a ring. In particular, as shown in Fig. 3, an attachment member 155 includes all of the features of described above with reference to attachment member 150 with the following exceptions. In particular, the attachment member 155 includes a tapered, longitudinally extending section 12 that is adapted and configured to guide the cannula 2 to a narrower point until further translation is not possible as the cannula reaches a generally cylindrical section 11 having a diameter substantially equal to that of the diameter of the lens 3 to inhibit obstruction of the field of view of the lens 3. Other structures for maintaining the gap between the lens 3 and the distal end 10 of the skirt member 4 are also accomplished. In an embodiment, at least one member extends from the cannula to maintain the gap. In an embodiment, skirt member 4 includes at least one rib that maintains the gap.

Diversion of the flow of organic matter and fluid, e.g., blood, away from the distal end 10 of the skirt member 4 is facilitated by the shape and structure of the attachment member. The skirt member 4 may have a bulbous or generally rounded shape to facilitate the flow of fluid, e.g., blood, over the skirt member 4 and away from the distal end 10 of the skirt member 4. In addition, at least a portion of the skirt member 4 may be formed from, or coated with, a hydrophobic material that repels fluid, e.g., blood, away from the skirt member 4, thereby inhibiting the flow of fluid to the lens 3. Parylene is an example of suitable hydrophobic material coating. At least a portion of the skirt member 4 may be formed from a flexible material.

Furthermore, maintaining the lens 3 at a distance "L" away from the distal end 10 inhibits the possibility of contamination of the lens 3 with organic matter and/or fluids. The greater the distance "L", the less likely the lens 3 will come into direct contact with internal bodily structures by creating a physical barrier. Moreover, the spacing between the surface of the lens 3 and the distal end 10 of the attachment member may generate a pressure gradient that inhibits the flow of fluid up through the lumen 3 and into contact with the lens 3.

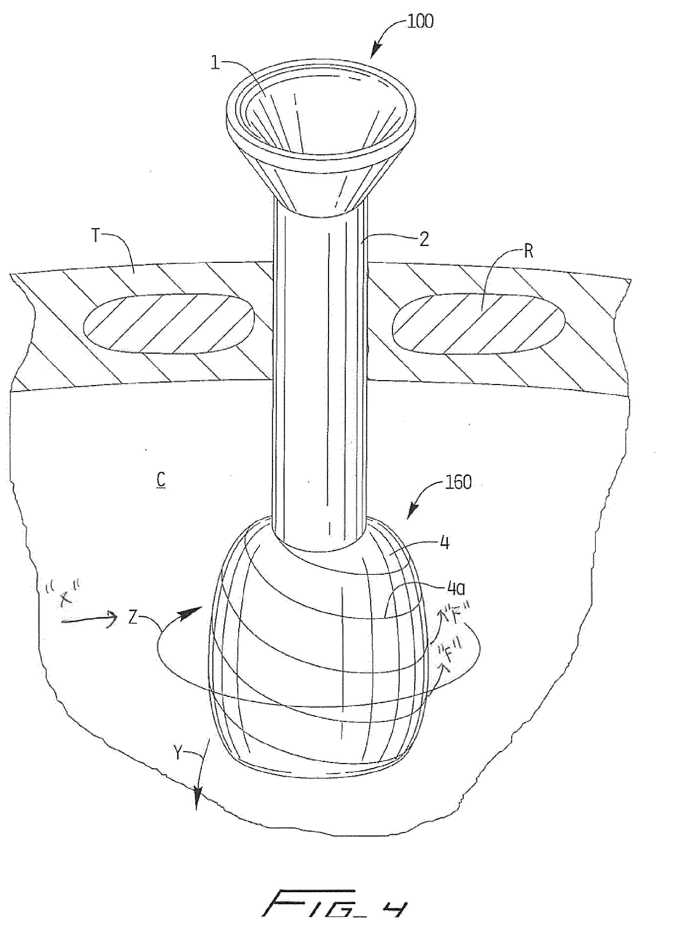

In an embodiment, an attachment member 160, as shown in Fig. 4, includes all of the features already discussed with respect to attachment member 150 and further includes channels 4a defined along the surface of the skirt member 4. The flow of fluid substantially perpendicular to the skirt member 4 as illustrated by directional arrow "X" is diverted by the channels 4a along directional arrows "F" driving the flow of fluid away from the distal end 10 of the skirt member 4. Moreover, an operator may rotate the cannula 2 along with the attachment member 160 in the direction indicated by arrow "Z". This in turn, generates a downward flow of fluid in the direction indicated by arrow "Y" as the channels 4a interact with fluid that may be along the surface of the skirt member 4.

In another aspect of the disclosure, a method for viewing a body cavity is disclosed. The method includes accessing an opening in a tissue layer, inserting a cannula within the opening, coupling an attachment member to a distal end of the cannula, and securing a viewing instrument within a passage of the cannula. The method may include any of the cannulas 2, attachment members 150, 155, 160, and viewing instruments 7 disclosed herein.

The method may further include rotating the attachment member to divert fluid away from a distal end of the attachment member. The method may further include creating an opening in a tissue layer.

While several embodiments of the disclosure have been shown in the drawings and/or discussed herein, it is not intended that the disclosure be limited thereto, as it is intended that the disclosure be as broad in scope as the art will allow and that the specification be read likewise. In particular, although the present disclosure relates to a thoracic scope, the present disclosure is applicable to any scope or viewing instrument used during a surgical procedure. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. A minimally invasive viewing instrument comprising:
   a cannula including a passage longitudinally disposed therethrough, the cannula including a distal end;
   a viewing instrument including a lens, the viewing instrument secured within the passage of the cannula; and
   an attachment member configured and adapted to be operatively coupled to the distal end of the cannula, the attachment member including:
      a skirt member including a proximal and a distal end, the skirt member including a lumen, the lumen being adapted to receive the cannula within a portion of the lumen while maintaining a gap between the lens and the distal end of the skirt member.
2. The minimally invasive viewing instrument of paragraph 1, wherein the lumen exerts a biasing force against the cannula.
3. The minimally invasive viewing instrument of paragraph 1 further comprising a ring disposed within the lumen, the ring being adapted and configured to frictionally engage the cannula to inhibit translation of the cannula.
4. The minimally invasive viewing instrument of paragraph 1, wherein the skirt member is formed from a material that repels fluid.
5. The minimally invasive viewing instrument of paragraph 1, wherein the skirt member defines a plurality of channels to propel and divert fluid away from the distal end of the skirt member.
6. The minimally invasive viewing instrument of paragraph 1, wherein the lumen tapers to a diameter that inhibits translation of the cannula.
7. A method for minimally invasive viewing of a body cavity comprising:
   accessing an opening in a tissue layer;
   inserting a cannula having a passage longitudinally disposed therethrough within the opening;
   coupling an attachment member to a distal end of the cannula, the attachment member including a skirt member having a proximal and a distal end, the skirt member including a lumen that receives the cannula; and
   securing a viewing instrument including a lens within the passage and extending into the lumen,
   the lumen maintaining a gap between the lens and the distal end of the skirt member.
8. The method of paragraph 7, wherein the lumen exerts a biasing force against the cannula.
9. The method of paragraph 7, wherein the attachment member further includes a ring disposed within the lumen, the ring frictionally engages the cannula when the cannula is received within the lumen to inhibit translation of the cannula.
10. The method of paragraph 7, wherein the skirt member is formed from a material that repels fluid.
11. The method of paragraph 7, wherein the skirt member defines a plurality of channels.
12. The method of paragraph 12, further including rotating the attachment member to divert fluid away from the distal end of the skirt member.
13. The method of paragraph 7, wherein the opening is a naturally occurring orifice.
14. The method of paragraph 7, wherein the opening is an incision created by the surgeon.

## Claims

1. A minimally invasive viewing instrument comprising:
a cannula including a passage longitudinally disposed therethrough, the cannula including a distal end;
a viewing instrument including a lens, the viewing instrument secured within the passage of the cannula; and
an attachment member configured and adapted to be operatively coupled to the distal end of the cannula, the attachment member including:
a skirt member including a proximal and a distal end, the skirt member including a lumen, the lumen being adapted to receive the cannula within a portion of the lumen while maintaining a gap between the lens and the distal end of the skirt member.

2. The minimally invasive viewing instrument of claim 1, wherein the lumen exerts a biasing force against the cannula.

3. The minimally invasive viewing instrument of claim 1 or claim 2 further comprising a ring disposed within the lumen, the ring being adapted and configured to frictionally engage the cannula to inhibit translation of the cannula.

4. The minimally invasive viewing instrument of any preceding claim, wherein the skirt member is formed from a material that repels fluid.

5. The minimally invasive viewing instrument of any preceding claim, wherein the skirt member defines a plurality of channels to propel and divert fluid away from the distal end of the skirt member.

6. The minimally invasive viewing instrument of any preceding claim, wherein the lumen tapers to a diameter that inhibits translation of the cannula.
